## Europäisches Patentamt
## European Patent Office
## Office européen des brevets

(19)

(11) Publication number: **0 269 218 B1**

# EUROPEAN PATENT SPECIFICATION

(12)

(45) Date of publication of patent specification: **06.03.91**  (51) Int. Cl.5: **C07C 263/20**

(21) Application number: **87308465.1**

(22) Date of filing: **24.09.87**

(54) Method for the recovery of isocyanates.

(30) Priority: **20.10.86 SE 8604441**

(43) Date of publication of application:
**01.06.88 Bulletin 88/22**

(45) Publication of the grant of the patent:
**06.03.91 Bulletin 91/10**

(84) Designated Contracting States:
**AT BE CH DE ES FR GB IT LI NL SE**

(56) References cited:
**FR-A- 2 320 938**
**US-A- 2 889 257**

(73) Proprietor: **Chematur Aktiebolag**
**Box 430**
**S-691 27 Karlskoga(SE)**

(72) Inventor: **Abrahamsson, Sören**
**Dragonvägen 18**
**Karlskoga(SE)**
Inventor: **Shuzhen, Wang**
**501 Chang Jiag Xi Lu**
**Shanghai(CN)**

(74) Representative: **Allam, Peter Clerk et al**
**LLOYD WISE, TREGEAR & CO. Norman**
**House 105-109 Strand**
**London WC2R 0AE(GB)**

## Description

The present invention relates to a method of recovering crude isocyanates such as TDI (toluendiisocyanate) and MDI (diphenyldiisocyanate) from mixtures containing said isocyanates and polymeric by-products. Such mixtures are e.g. the reaction masses in which the isocyanates are formed and the tarry material normally resulting from the distillation of the main part of the isocyanates from such reaction masses. The reaction mass in which an isocyanate is formed consists, apart for the isocyanate product, of polymeric by-products. While much of the isocyanate can be removed from such polymeric by-products by ordinary distillation, an appreciable quantity remains in the concentrated, viscous, tarry material which is formed as a result of this ordinary distillation. A complete removal of the isocyanate from this tarry material by simple distillation technique is difficult, as the tarry material is very sticky and highly viscous and has a poor heat transfer. Failure to recover this remaining amount of the isocyanate, which may be as high as 40% of the isocyanate originally formed, represents a significant economic loss.

Several different processes for improving the recovery of isocyanate product have been developed and practised. One such process is described in US-A-2889257, according to which the tarry mixture of the isocyanate and the polymeric by-products, received at the conclusion of the ordinary distillation of the main part of the isocyanate, is dropped into a naphthenic hydrocarbon oil which is held at a temperature of from 200° to 3l5°C, whereby the organic isocyanate is vaporized and the polymeric by-products converted into a filterable granular solid residue.

Whilst this prior process seems very simple in theory, in practice it has serious disadvantages. Thus, the use of a hot organic oil for the heat transfer introduces a high fire risk, which makes it necessary to keep the whole system under a blanket of inert gas, e.g. nitrogen. Moreover, the polymeric residue of this process, after the removal of the main part of the hot oil by centrifuging, is still contaminated with up to 20%− of the hot oil, which cannot be removed by ordinary centrifuging and which makes the residue very pyrophoric so that it ignites instantly if it is brought into contact with the air before it is cooled down. It is also known in the art that there are serious problems with solids build-up, agglomeration and plugging within the production devices built according to this prior patent.

Another prior process for improving the recovery of isocyanates is disclosed in FR-A-2320938. In this process, a thin (less than 10 mm) layer of the liquid distillation residue from which the recovery of further isocyanate product is desired, is spread onto a surface and then rapidly (less than one minute) heated, as by radiant heat and/or transference of heat from a hot metal surface, at a reduced pressure to a temperature of 100° to 200°C above the boiling point of the isocyanate. Next, the hot layer of distillation residue is slowly heated to increase its temperature to be at least 250°C above the boiling point of the isocyanate, and this heat treatment is continued until the isocyanate content of the distillation residue has been completely evaporated off and collected.

We have now observed that if a tarry mixture of an isocyanate and the polymeric by-products of the type mentioned above is very rapidly heated to within the range of 250° - 450°C, it quickly turns into a brittle, dry solid residue at the same time as the isocyanate is evaporated without noticeable decomposition.

We have further found that an excellent way to provide such a rapid heating of the tarry mixture is to use a direct contact, liquid heat transfer media, eg a molten metal with a suitable melting point such as lead, tin, zinc or soldering mixtures of lead, tin, zinc etc or a molten salt compound with a suitable melting point. The liquid heat transfer medium should have a suitable melting point and temperature, and it should also be non-combustible and heat stable as a melt. As mentioned above the liquid heat transfer media shall have a temperature of 250° - 450°C, so that, in addition to the metals mentioned above, molten salts such as for example, sodium nitrate, sodium nitrite and potassium nitrite, are also suitable heat transfer media for use in this invention.

The method according to the invention has several important advantages of which a few are mentioned below. Thus, the use of a heat-stable non combustible heat transfer medium, as is now possible, avoids the necessity to blanket with inert gas the whole production system, since it is now sufficient to blanket just the condenser where the vaporized isocyanate is recovered. Indeed, the whole process has become much less dangerous since the major fire hazards have been removed. The rapid evaporation of the isocyanate, when the tarry mixture meets the molten bath, breaks up the mixture, which is transformed into a residue in the form of porous, brittle solid particles which float on the surface of said bath, from where they may easily be removed. This residue, which consists of polymeric by-products may, if desired, be combusted in order to deliver heat to the process, although the residue as such is not particularly easy to ignite. We have thus suggested a rotating grate for this purpose.

The isocyanate-containing material may be added to the molten bath through a feeding system on or below the surface of said bath. If the feed material is delivered under the surface of the molten bath, then the feeding system should be particularly well cooled so that the isocyanate does not start to vaporize within the feeding system itself.

The invention is illustrated by the following laboratory example:

Example

Crude lead was melted in a porcelain cup with a gas flame. A tarry mixture containing 30% TDI 80-20 in admixture with polymeric by-products was dropped onto the surface of the molten lead. The TDI evaporated, leaving a residue of porous, brittle dry solid pellets on the surface.

A production plant suitable for performance of the method of this invention on a commercial seal is illustrated, somewhat schematically, in the accompanying drawing.

Referring now to the drawing, there is shown an evaporation chamber 1 containing a molten heat-stable and non-combustible bath 2 of a suitable metal, or mixture of metals, or a salt or a mixture of such salts, having a temperature of 250° to 450°C. Suitable metals are, for example, lead, tin or zinc, and suitable salts are, for example, sodium nitrate, sodium nitrite and potassium nitrate. The chamber 1 is also provided with a cooled injection nozzle 3 arranged below the surface of the melt 2.

The chamber 1 is further provided with a rotating agitator 4 arranged at the surface of said molten bath 2, a gas outlet pipe 16 which leads to a condenser 17, and a cell feeder 5 acting as a gas lock which leads to a combustion chamber 7. The combustion chamber 7 is provided with a rotating grate 8, a burner 9 for the start of the combustion in said chamber and a combustion gas outlet 11. The gas outlet II leads to a hot water boiler 12. The hot water boiler 12 is provided with an electric heater 13 which prevents the part 23 of the molten bath 2 which is collected at the bottom of the boiler 12 from freezing when no heat is delivered from the combustion chamber 7, and also a flue gas outlet 10. A hot water system 18 provided with a vapour separator 14 is also partly indicated on the drawing. A heat transfer system 19 including a circulating pump 15 between the boiler 12 and the chamber 1 is further indicated on the drawing. The condenser 17 is provided with an inlet 20 for an inert blanketing gas such as nitrogen and an outlet 21 for the condensed and recovered isocyanate.

In operation, the isocyanate-containing material such as the tar-like product described above is added below the surface of the melt 2 through the inlet nozzle 3. The isocyanate is rapidly evaporated on contact with the melt 2 and leaves the chamber 1 through the pipe 16, enters the condenser 17 where it is condensed, and leaves the condenser 17 as a liquid through the pipe 21. The polymeric residue of the feed floats to the surface of the bath 2 where the rotating agitator 4 pushes it towards the cell feeder 5. The cell feeder 5 delivers the residue, along with an amount of the melt 2 to the rotating grate 8 on which the residue is burned. The combustion gases which are formed leave the chamber 7 through the gas outlet 11 and enter the hot water boiler 12. The melt which leaves the bath 2 via the feeder 5 is collected at the bottom of the boiler 12 as the bath 23. Some of this melt is recirculated through the system 19 to the bath 2 in order to keep the temperature of the molten bath at a predetermined level.

The production system illustrated in this drawing thus produced crude isocyanate and useful heat, which, for example, can be used for the initial distillation of the isocyanate in the recovery process.

Claims

1. A method of recovering a crude organic isocyanate from a mixture containing said isocyanate and polymeric by-products, by rapid heating of said mixture whereby the isocyanate is vaporized and recovered by condensation, characterized in that said rapid heating is carried out by contacting said mixture with a heat-stable and non-combustible bath of molten metal or molten salt which is at a temperature within the range of 250° - 450°C.

2. A method according to Claim 1, wherein said mixture is brought into contact with said bath of molten metal or molten salt by introducing said mixture below the surface of the bath.

3. A method according to Claim 1, wherein said mixture is brought into contact with said bath of molten metal or molten salt by introducing said mixture onto the surface of the bath.

4. A method according to any preceding claim, wherein said molten bath consists of lead, tin or zinc, or of a mixture of two or more of said metals.

5. A method according to any one of Claims 1-3 wherein said molten bath consists of sodium nitrite, sodium nitrate or potassium nitrate, or of a mixture of two or more of said salts.

6. A method according to any preceding claim, wherein said organic isocyanate is toluene diisocyanate or diphenyldiisocyanate.

7. A method according to any preceding claim, wherein said mixture of isocyanate and polymeric by-products is a reaction mass resulting from the preparation of the isocyanate or the residue following distillation of isocyanate from such reaction mass.

8. A method according to any preceding claim, wherein the residue formed upon vaporization of said isocyanate is passed to a rotating grate and burned for the delivery of heat to a boiler and to said molten bath.

**Revendications**

1. Procédé pour récupérer un isocyanate organique brut à partir d'un mélange contenant ledit isocyanate et des sous-produits polymères, par chauffage rapide dudit mélange, ce qui provoque la vaporisation de l'isocyanate que l'on récupère par condensation, procédé caractérisé en ce qu'on effectue ledit chauffage rapide en mettant ledit mélange en contact avec un bain, stable à la chaleur et non combustible, d'un métal fondu ou d'un sel fondu qui est à une température comprise entre 250° C et 450° C.

2. Procédé selon la revendication 1, dans lequel ledit mélange est mis en contact avec ledit bain de métal fondu ou de sel fondu, par introduction dudit mélange audessous de la surface du bain.

3. Procédé selon la revendication 1, dans lequel ledit mélange est mis en contact avec ledit bain de métal fondu ou de sel fondu, par introduction dudit mélange à la surface du bain.

4. Procédé selon l'une quelconque des revendications précédentes, dans lequel ledit bain fondu consiste en du plomb, de l'étain ou du zinc ou en un mélange de deux ou de plus de deux de ces métaux.

5. Procédé selon l'une quelconque des revendications 1 à 3, dans lequel ledit métal fondu consiste en du nitrite de sodium, du nitrate de sodium ou du nitrate de potassium, ou bien en un mélange de deux ou de plus deux de ces sels.

6. Procédé selon l'une quelconque des revendications précédentes, dans lequel ledit isocyanate organique est du diisocyanate de toluène ou du diisocyanate de diphényle.

7. Procédé selon l'une quelconque des revendications précédentes, dans lequel ledit mélange d'isocyanate et de sous-produits polymères est une masse de réaction résultant de la préparation de l'isocyanate, ou est le résidu obtenu après distillation de l'isocyanate à partir d'une telle masse de réaction.

8. Procédé selon l'une quelconque des revendications précédentes, dans lequel on fait passer le résidu, formé par vaporisation dudit isocyanate, vers une grille rotative et on le fait brûler pour fournir de la chaleur à un bouilleur et audit bain fondu.

**Ansprüche**

1. Verfahren zum Gewinnen eines bröckeligen, organischen Isocyanats, aus einem Gemisch, das das Isocyanat und polymere Nebenprodukte enthält, durch schnelles Erhitzen des Gemisches, wobei das Isocyanat verdampft und durch Kondensation gewonnen wird, dadurch **gekennzeichnet,** daß das schnelle Erhitzen durch Kontaktieren des Gemisches mit einem wärmestabilen und nichtbrennbaren Bad eines geschmolzenen Metalls oder Salzes mit einer Temperatur im Bereich von 250-450° C erfolgt.

2. Verfahren nach Anspruch 1, dadurch **gekennzeichnet,** daß das Gemisch mit dem Bad geschmolzenen Metalls oder Salzes durch Einführen des Gemisches unterhalb der Oberfläche des Bades in Berührung gebracht wird.

3. Verfahren nach Anspruch 1, dadurch **gekennzeichnet,** daß das Gemisch mit dem Bad geschmolzenen Metalls oder Salzes durch Einführen des Gemisches auf die Oberfläche des Bades in Berührung gebracht wird.

4. Verfahren nach jedem der vorstehenden Ansprüche, dadurch **gekennzeichnet,** daß das Schmelzenbad aus Blei, Zinn oder Zink oder einem Gemisch aus zwei oder mehreren dieser Metalle besteht.

5. Verfahren nach jedem der Ansprüche 1 bis 3, dadurch **gekennzeichnet,** daß das Schmelzenbad aus Natriumnitrit, Natriumnitrat oder Kaliumnitrat, oder einem Gemisch aus zwei oder mehreren dieser Salze besteht.

6. Verfahren nach jedem der vorstehenden Ansprüche, dadurch **gekennzeichnet,** daß das organische Isocyanat Toluol-Diisocyanat oder Diphenyldiisocyanat ist.

7. verfahren nach jedem der vorstehenden Ansprüche, dadurch **gekennzeichnet,** daß das Gemisch aus Isocyanat und polymeren Nebenprodukten eine Reaktionsmasse ist, die aus der Herstellung des Isocyanats erfolgt, oder der Rückstand ist, der aus der Destillation des Isocyanats aus solcher Reaktionsmasse herrührt.

8. Verfahren nach jedem der vorstehenden Ansprüche, dadurch **gekennzeichnet,** daß der Rückstand der bei der Verdampfung des Isocyanats entsteht, durch einen Drehrost geleitet wird und für das Erzeugen von Wärme für einen Boiler und das Schmelzenbad, verbrannt wird.